(19) Europäisches Patentamt
European Patent Office
Office européen des brevets

(11) Numéro de publication : **0 130 935**
**B1**

(12) **FASCICULE DE BREVET EUROPÉEN**

(45) Date de publication du fascicule du brevet : **15.04.87**

(21) Numéro de dépôt : **84810156.4**

(22) Date de dépôt : **02.04.84**

(51) Int. Cl.⁴ : **C 08 G 69/08**, C 08 G 69/10, A 61 K 9/22// A61K47/00, A61L17/00

(54) **Polypeptide biodégradable et son utilisation pour le relargage progressif de médicaments.**

**Jointe à la demande no. 84901314.9/0150184 (numéro de dépôt/numéro de publication de la demande européenne) par décision du 27.08.86.**

(30) Priorité : **01.07.83 CH 3619/83**

(43) Date de publication de la demande : **09.01.85 Bulletin 85/02**

(45) Mention de la délivrance du brevet : **15.04.87 Bulletin 87/16**

(84) Etats contractants désignés : **AT BE CH DE FR GB IT LI LU NL SE**

(56) Documents cités :
**FR-A- 2 150 695**
**FR-A- 2 287 242**
**US-A- 4 356 166**
**CHEMICAL ABSTRACTS, vol. 96, no. 26, 28 juin 1982, page 369, no. 223180s, Columbus, Ohio, US; M. YOSHIDA et al.: "In vivo controlled release of testosterone from polypeptide-containing tablets" & KOBUNSHI RONBUNSHU 1982, 39(4), 285-91**

(73) Titulaire : **BATTELLE MEMORIAL INSTITUTE
7 route de Drize
CH-1227 Carouge/Genève (CH)**

(72) Inventeur : **Bichon, Daniel
16, rue de Vallard
F-74240 Gaillard (FR)**

(74) Mandataire : **Dousse, Blasco et al
7, route de Drize
CH-1227 Carouge/Genève (CH)**

**EP 0 130 935 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un polypeptide estérifié biodégradable nouveau dans lequel on peut incorporer des médicaments, ceux-ci étant, ensuite, libérés progressivement au fur et à mesure de la dégradation biochimique du polymère.

Depuis plusieurs années, on connaît des polymères biodégradables non toxiques pouvant servir de réservoir de médicaments et permettant la libération progressive contrôlée de ceux-ci dans l'organisme lors de la dégradation du polymère porteur. On trouve des informations générales sur de tels produits dans l'ouvrage : « Fundamental Aspects of Biocompatibility » par D.F. Williams, CRC Press (1981). Voir aussi US-A 4,093,709.

Parmi ces polymères, on cite plus particulièrement les polypeptides synthétiques (polyaminoacides) dont la structure est voisine de celle des protéines. Ces polypeptides sont biocompatibles et leurs produits de dégradation (acides aminés) sont résorbables par l'organisme. Ainsi Sidman et al. (J. Membr. Sci. (1980), 7 (3), 277-91) ont divulgué un copolymère d'acide glutamique et de $\gamma$-glutamate d'éthyle dont la vitesse de dégradation est fonction de la composition du copolymère (proportions molaires des segments estérifiés par rapport aux segments non estérifiés) et qui permet d'emmagasiner de nombreux produits médicamenteux, notamment des stéroïdes, des peptides, des produits anti-malaria, anti-cancer et autres. De tels polymères peuvent être utilisés sous forme de baguettes contenant, en mélange, le médicament désiré ou sous forme de capsules renfermant le médicament si celui-ci n'est pas miscible avec le polymère.

Malgré l'intérêt que présente le produit ci-dessus, on a continué à chercher un produit de qualités améliorées et présentant notamment les propriétés suivantes :

1. Excellente solubilité dans la plupart des solvants inoffensifs courants convenant aux médicaments (en effet, les dérivés connus de polyaminoacides ne sont, en général, solubles que dans certains solvants spéciaux (DMF, pyridine, $F_3CCOOH$) dont l'emploi est incommode pour les produits pharmaceutiques).

2. Thermoformabilité. Les polypeptides de synthèse actuellement connus ne sont en effet généralement pas miscibles avec les plastifiants biocompatibles usuels (polyalcoylène glycols) et, de ce fait, ils ne sont pas thermoplastiques.

3. Contrôle amélioré du processus de dégradation. En effet, la vitesse de dégradation des polypeptides synthétiques connus est liée de façon difficilement reproductible à leur structure chimique et notamment aux taux d'estérification. Ainsi, dans un cas donné (voir Sidman K.R., et al., PB 81-132136 NTIS (1980), p. 42) une variation du taux d'estérification de l'ordre de 10 % fait passer la vitesse de dégradation de 1 au centuple (voir également la référence : Sidman citée plus haut).

Le polymère de l'invention a permis de réaliser ces améliorations. Il s'agit d'un polypeptide estérifié de formule :

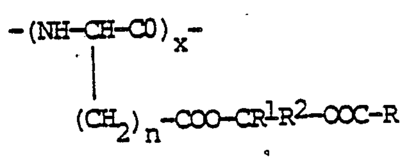

(I)

dans laquelle $R^1$ et $R^2$ sont des groupes alcoyles ou un atome d'hydrogène, R étant un reste aliphatique ou aromatique substitué ou non, ou bien $R^2$ est un atome d'hydrogène ou un groupe alcoyle et $R^1$ et R liés l'un à l'autre sont des groupes méthylène ou méthényle substitués ou non, n vaut 1 ou 2 et x est tel que la masse moléculaire soit d'au moins 5 000 D.

Comme on le voit de par la formule I, le polymère de l'invention est un polyaspartate ou polyglutamate estérifié par un dérivé d'acyloxyméthanol ($HO—CR^1R^2—OOCR$) dans lequel R est soit un reste organique quelconque, soit relié à $R^1$ de manière à former un cycle. Par « quelconque » on veut dire que la nature et la structure du groupe R n'est pas critique et que, pour l'instant, on n'a pas rencontré de cas où, R faisant partie de composés courants à fonction RCOO—, le composant correspondant de l'invention ne puisse être obtenu. On préfère, cependant, utiliser des composés dans lequel R est un groupe aliphatique ou aromatique substitué ou non substitué, les substituants étant choisis parmi les fonctions organiques biocompatibles. Parmi les groupes R préférés, on peut citer les groupes méthyle, éthyle, propyle, isopropyle, isobutyle, tert-butyle, phényle et benzyle. D'autres composés sont naturellement possibles, mais il est évident que pendant le temps limité dont a disposé l'inventeur il n'a pu les envisager tous.

Lorsque R et $R^1$ sont reliés ensemble de façon à réaliser une liaison carbone-carbone saturée ou non, ces atomes de carbone peuvent être, ou non, substitués par des restes aliphatiques ou aromatiques. On donne ci-dessous quelques exemples non limitatifs de tels groupes ester-lactal

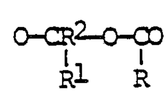

substitués ou non correspondant à la définition susmentionnée : groupe diméthylène $-CH_2-CH_2-$ ; groupe diméthylènethylène $-CH(CH_3)-CH(CH_3)-$ ; groupe vinylène $-CH{=}CH-$ ; groupe 1,2-phénylène

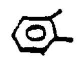

groupes cyclohexénylène

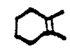

les groupes cyclopenténylène

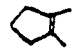

cyclopentadiénylène

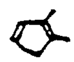

Le polymère de l'invention peut également se présenter sous la forme de copolymère avec d'autres polyaminoacides. Dans ce cas, on aura un copolymère de formule :

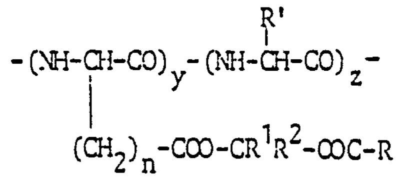

(II)

où R est un reste d'acide aminé estérifié ou non quelconque, les groupes R' des unités $-(NH-CHR'-CO)-$ pouvant être identiques ou différents dans la chaîne du copolymère, avec $y + z = x$, la valeur de x étant toujours choisie pour que le copolymère ait une masse moléculaire moyenne d'au moins 5 000 D. Bien entendu, si R' est identique au groupe $-(CH_2)-COO-CR^1R^2-OOC-R$, les n étant cependant différents (l'un d'entre eux valant 1 et l'autre 2), on aura un copolymère estérifié d'acide glutamique et aspartique. Cependant, en règle générale, on préfère pour R' avoir des groupements différents, tels que, par exemple : méthyle (alanine), isopropyle (valine), isobutyle (leucine et isoleucine) et benzyle (phénylalaline). En principe, tous les autres acides aminés, sont également possibles, quoique, pour des raisons évidentes, on n'ait pu les essayer tous. R' peut également désigner un reste d'acide glutamique ou aspartique non estérifié, ou estérifié partiellement par un alcool quelconque, par exemple MeOH ou EtOH, c'est-à-dire, par exemple, $-(CH_2)_n-COOH$ ou $-(CH_2)_n-COOMe$.

On pourra également avoir, indifféremment, des acides aminés de la série L ou D. Les acides aminés de la série L (ou naturels) sont les plus intéressants car les polypeptides les contenant sont dégradables par les enzymes (protéases) du corps humain, alors que les polypeptides constitués d'unités D ne le sont pas. On peut mettre à profit cette différence grâce à des copolymères comprenant des aminoacides D et L, ceci afin de disposer de polymères dont la vitesse de dégradation est modifiée.

Revenant à des considérations plus générales, il faut noter que la proportion molaire, dans le copolymère II, de l'autre polyaminoacide libre ou partiellement estérifié permet également dans une notable mesure de régler la vitesse de biodégradation du copolymère en fonction des agents présents dans l'organisme au site de destination du mélange de copolymère et du médicament à administrer, (c'est-à-dire dans l'organe où le médicament doit agir). Ainsi, par exemple, si le copolymère est un copolymère de polyglutamate I et de leucine, on choisira la préparation molaire relative des deux constituants en fonction de la vitesse relative de dégradation, au lieu considéré, du polyglutamate et de la polyleucine. En règle générale, le rapport z/y peut varier de 1 à 30, mais ces limites peuvent être dépassées si besoin est. Bien entendu, dans le cas où le groupe R' ne désigne pas un groupe de nature unique dans la chaîne du copolymère, c'est-à-dire, par exemple, lorsque l'un des R' désigne un reste d'acide aminé libre et qu'un autre R' désigne un reste d'amino-acide estérifié, on pourra, pour plus de commodité désigner les variantes de R' par les signes R'' et R'''. La formule générale d'un tel copolymère peut alors être schématisée comme suit :

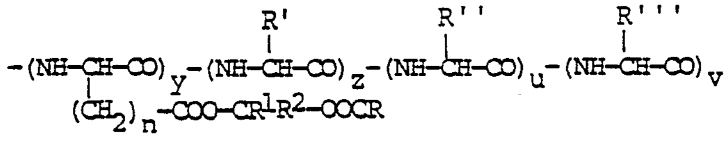

où la somme des indices y, z, u, et v est égale à x ; u et v peuvent être bien entendu nuls si le reste désigné par R' est de nature unique. Un cas typique où le copolymère présente des R' et R'' distincts est celui où

ces groupes désignent des restes d'acide glutamique et/ou aspartique estérifiés et non estérifiés, la formule schématique d'un tel polymère (dans le cas d'espèce, partiellement méthylé) se présentant comme suit :

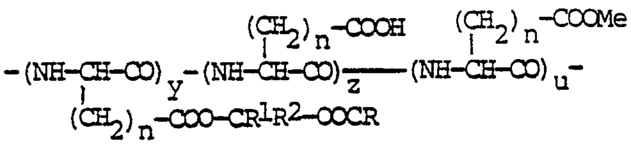

La nature du groupe R peut également influencer la vitesse de dégradation du polymère I. Ainsi, par exemple, si R est un groupe volumineux ou encombré (par exemple tert-butyle), la dégradation sera plus lente qu'avec un groupe méthyle ou éthyle.

Il est bien entendu que, du point de vue isomérie optique, les polymères de l'invention peuvent comprendre des éléments de configuration L ou D ou des mélanges racémiques ou, encore, des polymères où une des configurations domine. Les propriétés biochimiques de ces divers assemblages ne sont, bien évidemment, pas identiques, les polymères où dominent les formes naturelles (L) étant plus accessibles à la dégradation enzymatique. On peut donc en contrôler la dégradabilité en dosant, dans le copolymère les proportions relatives de l'une et l'autre forme.

Les polymères I et copolymère II sont insolubles dans l'eau et généralement solubles dans un ou plusieurs des solvants usuels tels que l'acétone, le méthyléthyl cétone (MEK), le tétrahydrofurane (THF), le dioxanne, l'acétate d'éthyle, le monoglyme et le diglyme, ce qui permet leur transformation aisée en billes, bâtonnets, fibres, filaments, microcapsules et films. Les polymères I et II suivant leur structure peuvent être insolubles ou solubles dans les solvants chlorés, par exemple le chloroforme. Dans certains cas d'insolubilité dans le chloroforme, on peut y remédier si on ajoute à un tel solvant un peu d'acétone. Cette faculté de dissolution dans de nombreux solvants miscibles ou non miscibles à l'eau les rend également compatibles sans autre avec de nombreux médicaments liquides ou solubles dans les mêmes solvants. Ainsi, par exemple, on peut, pour encapsuler un produit hydrosoluble dans des microbilles de polymère, utiliser la technique connue consistant à disperser une solution aqueuse du médicament dans une solution de polymère, celle-ci comprenant un solvant non miscible à l'eau, puis à évaporer ce solvant de manière que se forment les capsules solides de polymère.

Par ailleurs, suivant sa structure, le polymère I est très souvent parfaitement compatible avec les polyalcoylène glycols (polyéthylène glycol et polypropylène glycol), ce qui permet d'utiliser ces polyéthers glycol comme plastifiants du polymère I et de fournir ainsi un mélange homogène de bas point de fusion. On peut facilement incorporer toute une gamme de médicaments thermolabiles à un tel mélange (fusion à des températures de l'ordre de 40 à 60 °C) et en obtenir des granulés ou des microcapsules. De plus, la présence de polyalcoylène glycols très hydrophiles permet d'augmenter la susceptibilité du polymère et du copolymère aux liquides aqueux biologiques et faciliter leur dégradation enzymatique in situ. Il est à remarquer que les polypeptides de synthèse connus ne possèdent pas ces propriétés favorables de solubilité et de compatibilité avec les PEG. Ainsi, par exemple, pour former des films d'acide polyglutamique présentant une résistance mécanique appréciable et une certaine insolubilité dans l'eau, on doit utiliser des solutions dans des solvants relativement malaisés à manipuler et peu appréciés en pharmacie tels que diméthyl formamide (DMF) et acides dichloracétique (DCA) et trifluoroacétique (TFA). Les films d'acide polyglutamique obtenus à partir de solutions aqueuses, (à pH 7,4, c'est-à-dire où l'acide est au moins en partie sous forme salifiée) n'ont aucune résistance mécanique et sont rapidement dissous dans l'eau, ce qui rend le polymère entièrement impropre en tant que support de médicament retard au sens de la présente invention. Il en est d'ailleurs de même des mélanges acide polyglutamique-polyéthylène glycol qui sont instantanément solubles dans l'eau.

La biodégradation du polymère I peut être schématisée comme suit :

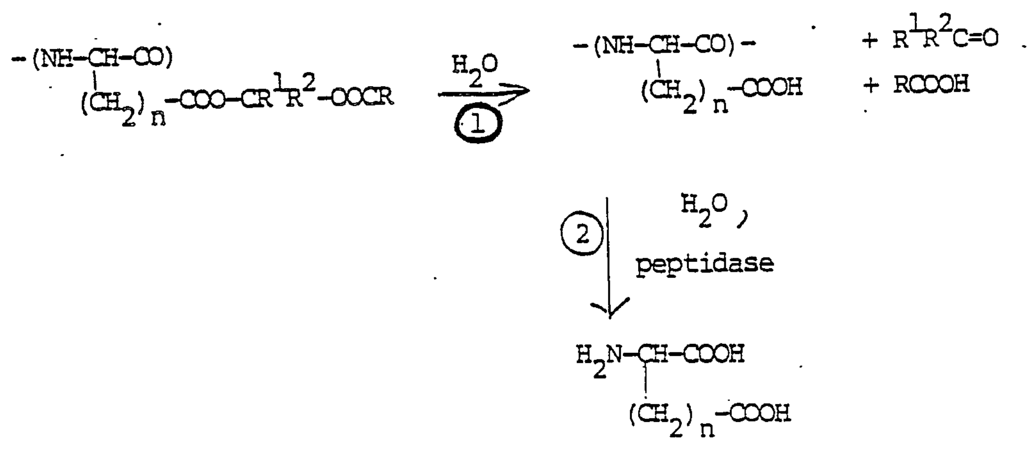

La réaction (2) est consécutive à la réaction (1) et, de ce fait, la biodégradation du polymère sera d'autant plus rapide que la vitesse d'hydrolyse de l'ester lactal ou acyloxyalcoylique est plus grande. Le mécanisme d'hydrolyse de ce type d'esters est connu en soi et a été mis en évidence lors de l'étude d'esters de l'ampicilline (par exemple la pivampicilline), voir à ce sujet la référence suivante : « Design of biopharmaceutical Properties through Prodrugs and Analogs », B. Roche, éditeur, American Pharmaceutical Association (ISBN 0-917330-16-1), page 212 et 354 (1977). Il est à noter que lorsque $R^2$ est l'hydrogène et $R^1$ un alcoyle (par exemple méthyle), le composé résultant de la réaction (1) est un aldéhyde $R^1$—CHO (par exemple l'acétaldéhyde), de tels aldéhydes étant biologiquement plus avantageux que le métaldéhyde obtenu lorsque $R^1=R^2=H$ pour des raisons de toxicité. En ce qui concerne cet aspect de l'invention, les produits résultant de la réaction (1) lorsque R et $R^1$ sont reliés l'un à l'autre (des céto-acides ou acide-aldéhydes) sont également très avantageux en raison de leur toxicité négligeable. Ainsi, si l'association R—$R^1$ correspond aux groupes éthylène ou 1,2-phénylène, les produits de dégradation seront, respectivement, les acides 3-formyl-propionique et O-formyl-benzoïque lentement éliminés par l'organisme sans réactions secondaires.

On peut préparer le polymère I par estérification directe d'un sel du polyaminoacide correspondant avec un halogénure d'acyloxyméthyle (X—$CR^1R^2$—OCO—R (III)) ou X peut être le chlore, le brome ou l'iode. Le sel de polyaminoacide est, de préférence, un sel d'amine tertiaire (par exemple de tributylamine ou de triéthylamine). Une telle technique est connue en soi de par W. V. Dachne ; J. Méd. chem. *13*, 607-12 (1971). Par ailleurs, la synthèse des composés III (X = Cl) est connue (voir Z. H. Ulich, J. A. C. S. *43*, 662 (1921)) et consiste à faire réagir le chlorure d'acide RCOCl avec le formaldéhyde en présence d'une quantité catalytique de $ZnCl_2$ anhydre.

Le polyaminoacide ou co-polyaminoacide dont l'estérification fournit le polymère I ou le copolymère II s'obtient facilement par les moyens habituels comprenant l'estérification par un alcool inférieur du carboxyle latéral d'un acide de formule

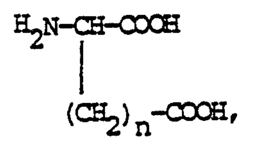

la transformation de l'ester en N-carboxyanhydride correspondant (NCA) par le phosgène en milieu dioxane ou THF, la polymérisation du NCA en polyaminoacide estérifié et l'hydrolyse du groupe ester protecteur en milieu alcalin ou par l'acide trifluoroacétique. De telles méthodes sont connues en soi (voir par exemple Encyclopedia of Polymer Science and Technology ; N-carboxyanhydrides, vol. II, page 837). Lorsqu'on désire parvenir à un copolymère ou R' désigne un carboxyle latéral partiellement estérifié ($R'=-(CH_2)_n-COOH$ et $R''=-(CH_2)_n-COOAlk$) on prendra soin que l'hydrolyse du groupe ester protecteur ne soit que partielle. Ainsi, par exemple, le produit de départ qu'on estérifiera avec le composé $XCR^1R^2$—OCOR sera un copolymère d'acide $H_2N-CH[(CH_2)_n-COOH]-COOH$ et d'ester $NH_2-CH[(CH_2)_n-COOAlk]-COOH$.

Le polymère I et le copolymère II sont utilisables comme réservoir de médicaments de diverses manières. Ainsi, par exemple, on peut employer les présents polymères I et copolymères II pour fabriquer des microcapsules contenant un médicament. De telles microcapsules comprennent une membrane polymérique et contiennent une solution aqueuse ou huileuse dans laquelle le médicament est en suspension ou en solution. On peut également fabriquer des microsphères, c'est-à-dire des particules solides ou billes contenant le médicament à l'état dispersé ou à l'état de solution solide dans la matrice de polymère. On peut également fabriquer des produits microporeux dénommés microéponges. En général, on pourra mettre en œuvre, au moyen des présents polymères, toutes les techniques de fabrication de médicaments retard, c'est-à-dire ayant la propriété de relâcher (relarguer) le médicament de manière prolongée au fur et à mesure de la dégradation du support. On trouvera une description de ces techniques dans les ouvrages suivants : « Biodegradables and Delivery Systems for Contraception », Mafez E. S. E., MTP Press limited (1980) ; « Controlled Release Technologies = Methods, Theory and Applications » Vol. 1 et 11, A. F. Kydonieus, CRC Press (1980) et « Microencapsulation — New Techniques and Applications » par Tamotsu Kondo, Techno. Inc. (1979) Japan. La solubilité des présents polymères dans de nombreux solvants, miscibles à l'eau ou non, est un avantage pour leur application selon les techniques décrites dans ces références. Il est également possible de préparer des fils constitués de ces polymères en extrudant une solution de ceux-ci dans une filière et en précipitant le fil soit par évaporation, soit par un bain de non-solvant, selon les techniques habituelles de filage. Des filaments préparés ainsi peuvent être tricotés, noués ou tissés pour former des sutures, des ligatures ou des structures tubulaires pouvant servir d'artères artificielles, de veines, de conduits ou d'organes internes à fonctionnement temporaire. Les polymères de l'invention peuvent également servir, soit directement, soit en mélange avec un plastifiant, à la fabrication de films ou de prothèses chirurgicales servant, par exemple, à la consolidation d'os fracturés, comme des agrafes, des aiguilles, des vis, des plaques de renforcement et des tampons ...., ces matériaux pouvant être réalisés par coulage ou moulage de solution, thermoformage ou par usinage de blocs de polymère solides. De telles prothèses étant résorbables, elles

sont progressivement éliminées dans l'organisme et il n'est alors plus nécessaire de prévoir, comme on le fait actuellement, une nouvelle opération pour enlever le matériau de renforcement et de consolidation.

Bien entendu, la composition exacte du polymère ou copolymère utilisé est à régler, en fonction des vitesses de dégradation et des caractéristiques d'absorption in vivo, suivant la nature de la prothèse envisagée.

Les exemples qui suivent illustrent l'invention.

Exemple 1

Copolymère de polyglutamate de benzoyloxyméthyle, d'acide glutamique et de glutamate de méthyle.

On a préparé de l'acide polyglutamique partiellement méthylé à partir du N-carboxyanhydride de γ-glutamate de méthyle dissous dans le chlorure de méthylène ; on a utilisé la triéthylamine comme initiateur de polymérisation (A/1 = 100). On a ensuite précipité le polymère par adjonction de méthanol, puis on l'a séché sous vide. On a redissous le solide dans de l'acide trifluoracétique (TFA) de manière à réaliser une solution à 5 % en poids et on a ajouté goutte à goutte en agitant vigoureusement un volume d'eau distillée suffisant pour que la solution finale contienne des volumes égaux d'eau et de TFA. On a maintenu encore 12-15 h en agitation à température ambiante (solution visqueuse) après quoi on a versé le tout sur de l'eau distillée en grand excès, ce qui conduit à la précipitation d'un copolymère de glutamate de méthyle et d'acide polyglutamique, l'hydrolyse de l'ester méthylique ayant atteint environ 60-70 %. On a filtré ce copolymère et on l'a séché. On a contrôlé les proportions relatives de groupes COOH libres et estérifiées du copolymère ainsi obtenu par analyse RMN dans le TFA (intégration de la bande ester méthylique $—O—CH_3$ à 3,95 ppm). On a effectué une analyse de poids moléculaire par GPC (chromatographie par perméation de gel) dans le DMF sur colonne Dupont ZORBAX PSM bimodale (étalonnage au polystyrène) ; on a mesuré un poids moléculaire moyen de Mn (number average molecular weight) de 226 000 ; et une dispersion (Mw/Mn) de 1,75.

On a dissous 1,22 g de copolymère dans 45 ml de DMF et on a ajouté 3,5 g de tributylamine. On a ensuite ajouté goutte à goutte 3,25 g de benzoate de chlorométhyle préparé suivant Ulich, J. A. C. S. *43*, 662 (1921) et on a continué à agiter 48 heures à température ambiante. On a alors ajouté 50 ml d'eau, ce qui a causé la précipitation d'un solide incolore qu'on a filtré, séché et purifié par successivement, dissolution dans l'acétone et précipitation à l'éther, puis dissolution dans l'acétone et précipitation à l'eau. Par analyse RMN du produit dans le TFA, on a constaté la présente des résonances suivantes :

δ = 7,8 ppm (4H, aromatiques) ; 8,4 ppm (1H, amido) ; 6,5 ppm (2H ; $O—CH_2—O$) ; 2-3 ppm (complexe, 4H, $β—CH_2$) ; ($γ—CH_2$) 5 ppm (1H, α—CH) ; 3,95 ppm ($CH_3$ ester). L'intégration du spectre a montré que l'estérification des fonctions acides libres par le chlorométhylbenzoate était de l'ordre de 60 % et que dans la formule du copolymère telle que représentée ci-dessous :

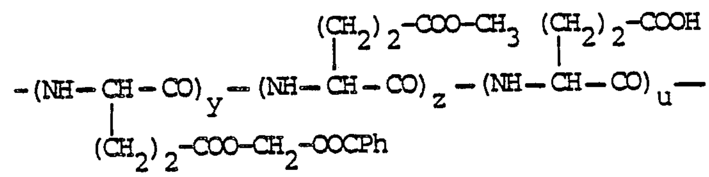

on a, pour les indices, les valeurs suivantes : y = 0,6 ; z = 0,33 et u = 0,07.

Ce polymère est insoluble dans l'eau, $CH_2Cl_2$, $CHCl_3$ et l'éther ; il gonfle sans se dissoudre dans le méthanol et l'éthanol ; il se dissout facilement dans les solvants suivants : acétone, méthyl-éthyl cétone ; THF, AcOEt DMF, TFA, acide dichloracétique (DCA). On peut préparer des films minces de polymère en étalant sur des substrats des couches de ces solutions et en les laissant évaporer.

Exemple 2

Copolymère d'acide glutamique partiellement méthylé et de glutamate de pivaloyloxyéthyle.

Suivant le processus décrit à l'exemple précédent on a préparé un copolymère d'acide glutamique et de glutamate de méthyle par hydrolyse ménagée du polyglutamate de méthyle dans une solution aqueuse de TFA. Le copolymère obtenu avait la formule

$$Glu(OMe)_{0,25}—Glu(OH)_{0,75}$$

On a dissous 2 g de ce copolymère dans 50 ml de DMF sec et on a ajouté goutte à goutte à cette solution 5,74 g (0,031 mole) de tributylamine et 5,09 g (0,031 mole) de pivalate d'α-chloroéthyle (préparé selon la référence citée à l'exemple précédent, par action du chlorure de pivaloyle sur le paraldéhyde). Après 4 jours d'agitation à température ordinaire, on a dilué par un excès d'eau ce qui a eu pour effet de précipiter le polymère sous forme d'une poudre incolore qu'on a redissoute, après séchage, dans de l'acétone et reprécipitée à l'éther de pétrole. On a procédé à encore deux étapes de purification par

dissolution dans l'acétone et reprécipitation à l'éther de pétrole, ce qui a finalement fourni 1,8 g de produit. Par analyse RMN, on a pu établir que le copolymère répondait à la formule suivante :

$$Glu(OMe)_{0,25}—Glu(OH)_{0,6}—Glu(O—CH(CH_3)—OOCt.Bu)_{0,15}$$

Les résultats d'analyse étaient les suivants :
δ = 1,35 ppm (s,tert-butyle) ; = 1,7-1,8 ppm (d, —CH(<u>CH$_3$</u>)—)
δ = 6,8 ppm (—<u>CH</u>(CH$_3$)—)

Ce polymère est soluble dans les solvants mentionnés à l'exemple précédent à l'exception du chloroforme.

Exemple 3

On a répété les opérations décrites à l'exemple précédent en utilisant, cette fois, un copolymère de formule $Glu(OMe)_{0,5}—Glu(OH)_{0,5}$ obtenu, comme décrit plus haut par hydrolyse ménagée du polyglutamate de méthyle, le temps de celle-ci étant limité à 6 h. On a établi que le copolymère correspondait bien à la formule susmentionnée en comparant les valeurs d'intégration des protons en RMN, le signal à 3,95 ppm ($O—CH_3$) et celui à 5 ppm (α—CH).

On a fait réagir 1 g de ce copolymère avec 2 équivalents de pivalate d'α-chloroéthyle pendant 3 jours puis précipitation de sa solution dans le DMF avec de l'eau. Après purification comme décrit à l'exemple précédent, on a établi par analyse RMN, comme décrit plus haut, que la formule du copolymère s'établissait ainsi :

$$Glu(OMe)_{0,5}—Glu(OH)_{0,33}—Glu(OCH(CH_3)—OCO—t.Bu)_{0,17}$$

Ce copolymère est soluble dans les solvants précités et également dans $CHCl_3$. Il est insoluble dans le polyéthylèneglycol 400.

Exemple 4

On a préparé le 3-bromophtalide de formule

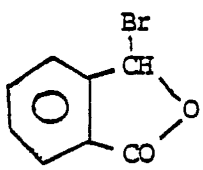

(3-bromo-1 (3H) isobenzofuranne)

par bromination du phtalide d'après le document GB-A-1,364,672, p. 5.

On a dissous 2 g d'acide polyglutamique et 5,74 g (2 equiv.) de tributylamine dans 35 ml de DMF sec et, à ce mélange, on a ajouté 16,7 g du bromophtalide ci-dessus. Après quelques minutes, on a constaté un épaississement important de la solution (gel) qu'on a redissous par adjonction de 5 ml d'eau. Après 6 jours d'agitation à température ambiante, on a ajouté 500 ml d'éthanol, ce qui a provoqué la précipitation d'un produit pulvérulent incolore qu'on a filtré, essoré, rincé et séché. On a redissous le produit dans 40 ml de $CHCl_3$ et on l'a purifié en le précipitant à l'éther. Rendement 2,77 g (68 %). Par analyse RMN on a obtenu les résultats suivants : δ = 8 ppm (4H, Bz + 1H, peptid.) ; δ = 7,7 ppm (CH lactonique) ; δ = 5 ppm (α—CH) ; δ = 2-3 ppm (m, $—CH_2—CH_2—$).

En comparant, après intégration, le rapport entre le proton (α—CH) et les protons aromatiques on a établi que le rendement de l'estérification était de 90 %. La formule du produit s'établit donc ainsi :

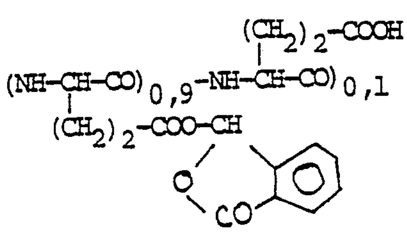

Ce produit, très soluble dans $CHCl_3$, est insoluble dans l'eau, l'alcool, le polyéthylène glycol et l'acétone pur. Il est soluble dans un mélange d'acétone —$CHCL_3$.

On a préparé un film sec de quelques um de ce polymère au moyen d'une solution chloroformique étalée sur une plaquette de verre et évaporée. On a placé ce film dans un récipient contenant une solution 0,1 N tampon à pH 9,5 et on a constaté une lente dégradation du polymère qu'on a observée, jour après jour, par augmentation de l'absorption UV de la solution (280 nm) due à la présence du phtalide qui se forme progressivement dans la solution tampon.

Exemple 5

On dissout 10 g d'un copolymère 1/1 de poly(γ-méthyl-L-glutamate) et de poly(pivaloyloxyméthyl-L-glutamate) préparé selon l'exemple 2 dans 75 ml de chloroforme et on ajoute 5 g d'indométhacine (Sigma). On obtient ainsi une solution visqueuse, de couleur jaune et on verse celle-ci goutte à goutte, sous forte agitation, dans 1 litre d'eau distillée contenant 0,1 % de dodécylsulfate de sodium (SDS). On maintient cette solution sous agitation à 40 °C jusqu'à évaporation complète du chloroforme, ce qui fournit une dispersion de microsphères. On filtre celles-ci et on les sépare en lots de différentes tailles à l'aide de tamis calibrés. Ces microsphères se dissolvent en milieu isothermique (pH 7,5) et, au cours d'une période prolongée libèrent le médicament qu'elles contiennent, la vitesse de « relargage » variant selon leur taille moyenne. Lorsqu'on injecte une telle suspension dans un tissu présentant une affection inflammatoire, l'action du médicament se prolonge bien au-delà de celle d'un médicament conventionnel à base d'indométhacine.

Exemple 6

Polyglutamate de pivaloyloxyméthyle

On a préparé de l'acide polyglutamique à partir du N-carboxyanhydride de γ-glutamate de méthyle dissous dans le chlorure de méthylène ; on a utilisé la triéthylamine comme initiateur de polymérisation (A/I = 100). On a ensuite précipité le polymère par adjonction de méthanol, puis on l'a séché sous vide. On a redissous le solide dans de l'acide trifluoracétique (TFA) de manière à réaliser une solution à 5 % en poids et on a ajouté goutte à goutte en agitant vigoureusement un volume d'eau distillée suffisant pour que la solution finale contienne des volumes égaux d'eau et de TFA. On a maintenu encore 24 h en agitation à température ambiante (solution visqueuse) après quoi on a versé le tout sur de l'eau distillée en grand excès, ce qui conduit à la précipitation de l'acide polyglutamique. On a filtré cet acide et on l'a séché. On a contrôlé la pureté de l'acide ainsi obtenu par analyse RMN dans le TFA (absence de la bande ester méthylique $-O-CH_3$ à 4,5 ppm). On a effectué une analyse de poids moléculaire par GPC (chromatographie par perméation de gel) dans le DMF sur colonne DUPONT ZORBAX PSM bimodale (étalonnage au polystyrène) et on a mesuré les valeurs suivantes $\overline{M}_n$ = 226 000 ; $\overline{M}_W$ = 397 000 ; $\overline{M}_Z$ = 723 000 ; dispersivité = 1,75.

On a dissous le polyacide dans le DMF à raison de 5 % en poids et, à 50 ml de cette solution, on a ajouté 4 ml d'eau et 4,04 g (0,04 mole) de triéthylamine. On observe d'abord une précipitation du polyglutamate de triéthylamine, ce sel se redissolvant ensuite par agitation lorsqu'on ajoute l'eau. Lorsque tout a été dissous, on a ajouté goutte à goutte 6,02 g de pivalate de chlorométhyle (Fluka AG) et on a continué à agiter 48 heures à température ambiante. On a alors ajouté 50 ml d'eau, ce qui a causé la précipitation d'un solide incolore qu'on a filtré, séché et purifié par successivement, dissolution dans l'acétone et précipitation à l'éther, puis dissolution dans l'acétone et précipitation à l'eau. Par analyse RMN du produit dans le TFA, on a constaté la présence d'un pic tert-butyle à 1,35 ppm. L'intégration du spectre a montré que l'estérification de l'acide polyglutamique par le chlorométhylpivalate était totale.

Le poids moléculaire a été déterminé comme ci-dessus et a fourni les résultats suivants : $M_n$ = 30 480 ; $M_w$ = 708 000 ; $M_z$ = 297 000 ; dispersivité = 3,54 (viscosité relative $\eta$ = 1,57, C = 0,2 g/dl dans le DMF). Rendement final 51 %.

Ce polymère est insoluble dans l'eau, $CH_2Cl_2$, $CHCl_3$ et l'éther ; il gonfle sans se dissoudre dans le méthanol et l'éthanol ; il se dissout facilement dans les solvants suivants : acétone, méthyl-éthyl cétone : THF, AcOEt/DMF, TFA, acide dichloracétique (DCA). On peut préparer des films minces de polymère en étalant sur des substrats des couches de ces solutions et en les laissant évaporer. Le spectre IR confirme également la structure proposée (forte bande à 1 740 $cm^{-1}$).

Exemple 7

On a préparé un film mince du polymère obtenu suivant l'Exemple 6 en étalant une couche de solution à 10 % dans l'acétone et en la laissant sécher quelques heures à température ordinaire à l'air.

On a ensuite immergé plusieurs échantillons de ce film dans une solution à 1 % de pancréatine dans du tampon phosphate 0,1 M (pH 7,5) et on a abandonné ces échantillons à température ambiante pendant 1 à 8 jours. On a constaté, après 1 jour, une perte notable de résistance mécanique (env. 50 %) et après 2 jours. une disparition quasi totale de cette résistance (l'échantillon s'est fragmenté en morceaux). Après 8 jours. l'échantillon s'était entièrement dégradé et n'était plus visible. Des résultats analogues ont été observés en utilisant de l'estérase ou de la leucinaminopeptidase.

Exemple 8

Préparation d'un copolymère ester pivaloyloxyméthylglutamique et leucine.

On a opéré comme dans l'exemple 6, mais en partant d'un copolymère 85/15 d'acide polyglutamique et de leucine et on a obtenu un copolymère 85/15 de pivaloyloxyméthyl glutamate/leucine. Le spectre RMN du polymère en solution dans le TFA montre que 100 % des groupes carboxyliques latéraux de l'acide polyglutamique sont estérifiés par le chlorométhylpivalate. Le polymère est également soluble dans l'acétone, la MEK, la DMF, le THF. Il gonfle dans les alcools, sans se dissoudre. Spectre RMN : $\delta$ = 1,35 ppm, 9 protons tert-butyle ; $\delta$ = 1, 0, 6 protons isobutyle. Intégration : rapport des groupements 85 : 15.

Exemple 9

Préparation d'un polymère ester d'isobutyloxyméthylglutamate.

On a effectué la synthèse en partant d'acide polyglutamique préparé comme décrit dans l'Exemple 6 ; on a dissous ce polymère dans du DMF préalablement séché sur tamis moléculaire de manière à obtenir une solution à 3,2 %. A 20 g de cette solution, on a ajouté 1,83 g de tributylamine (2 équivalents). Contrairement à ce qui a été observé avec la triéthylamine, il n'est pas nécessaire d'ajouter de l'eau pour que la dissolution s'effectue. On ajoute ensuite goutte à goutte 1,36 g d'ester chlorométhylique de l'acide isobutyrique (préparé suivant (Ulich, J.A.C.S. *43,* 662, (1921) (Eb. 65 °C/48 Torr) à la solution de polyglutamate de tributylamine et on laisse 3 jours sous agitation à température ambiante. Le mélange réactionnel est ensuite précipité par l'eau, redissous dans l'acétone, reprécipité par l'hexane, redissous dans l'acétone et précipité à nouveau dans l'eau. L'analyse RMN du polymère séché montre la présence de pics isobutyle ($\delta$ = 1,15 ppm, doublet) caractéristiques du produit recherché et, par intégration, 90 % de substitution (obtenu 1,03 g, 90 %). Le polymère est également soluble dans l'acétone, la MEK, le THF, le DMF. Le spectre infrarouge d'un film coulé à partir d'une solution de TFA, ainsi que l'analyse élémentaire correspondent à la structure proposée.

Exemple 10

Préparation pharmaceutique à base de polyglutamate de pivaloyloxyméthyle plastifié au PEG :

On dissout 5 g de poly-pivaloyloxyméthylglutamate et 5 g de polyéthylèneglycol 600 (Fluka, AG) et 2 g d'indométhacine (Sigma) dans 100 ml d'acétone. On coule un film à partir de cette solution sur une plaque de verre et on laisse évaporer le solvant. Le film obtenu, chauffé à 50 °C, fond et peut être moulé sous forme de capsules qui, une fois refroidies et plongées dans une solution aqueuse isotonique, laissent diffuser de l'indométhacine dans le milieu.

Exemple 11

Synthèse de poly-acétoxyméthylglutamate

On a effectué la synthèse comme dans l'exemple 9, mais en partant de chlorométhylacétate.

Le polymère obtenu est également soluble dans l'acétone. Son spectre RHN montre la présence d'un pic à 3,2 ppm, caractéristique du groupe acétyle (3 protons). Le polymère se dissout dans la soude caustique aqueuse N/100 en s'hydrolysant en quelques minutes.

Exemple 12

Biodégradation in vitro du poly-pivaloyloxyméthylglutamate

On a préparé, suivant la méthode de l'Exemple 6, du poly-pivaloyloxyméthylglutamate dont le groupement méthyle est marqué au $^{14}C$. On a pour cela synthétisé du chlorométhylpivalate en faisant réagir du chlorure de pivaloyle sur du paraformaldéhyde marqué au $^{14}C$, selon la technique décrite dans J.A.C.S., 89 (21), 5442, (1967).

L'activité spécifique du polymère, mesurée par combustion est de 3 µCie/g. On a préparé avec ce polymère des films de 3 × 3 cm à partir de solutions dans l'acétone ou dans le TFA. Les films obtenus sont trempés soit dans des solutions enzymatiques de Leucineaminopeptidase de rein de porc (Sigma, 3,7 Unités/ml, tampon 0,1 tris, 5 mM $MgCl_2$, pH 8,4), soit d'estérase de foie de porc, (Sigma, 11,6 Unités/ml, tampon 0,1 M Tris, pH 7,5). On mesure la vitesse de dégradation en observant d'une part l'aspect du polymère et, d'autre part, en comptant le degré de radioactivité acquise par la solution. On a renouvelé les solutions enzymatiques chaque jour. On a obtenu les résultats suivants :

| temps d'incubation | Esterase (pH 7,5) | (pH 8,4) Leucine Aminopeptidase | |
|---|---|---|---|
| film coulé à partir d'une solution de: | Acétone | Acétone | TFA |
| 1 jour | film opaque, 3,5 % hydrolyse | film opaque, 13,6 % hydrolyse | film opaque, 12,6 % hydrolyse |
| 2 jours | film blanchâtre, soufflé, 9 % hydrolyse | film opaque, 29 % hydrolyse | film très déformé, 46,8 % hydrolyse |
| 4 jours | film blanchâtre, soufflé, 17 % hydrolyse | film opaque, soufflé, 52 % hydrolyse | film désagrégé, dissous, quelques débris solides ; 85 % hydrolyse |
| 5 jours | film gonflé, 24 % hydrolyse | film commence à se casser, 71 % hydrolyse | |
| 7 jours | film gelatineux. 37 % hydrolyse | film entièrement désagrégé, 85 % hydrolyse | |

Exemple 13

On dissout du poly-pivaloyloxyméthyl glutamate dans de l'acétone et on ajoute 10 % en poids du polymère de polyéthylèneglycol 600. On coule la solution sur une plaque de Téflon et on laisse évaporer le solvant. Le film obtenu est translucide et peut être soudé thermiquement vers 120 °C.

Exemple 14

On a préparé un copolymère d'acide aspartique et de leucine (voir Polymer 16, 735 (1975)) en copolymérisant du β-benzylaspartate NCA et de la leucine NCA (N-carboxyanhydrides) en proportions équimoléculaires. On transestérifie les groupes benzyl ester par le méthanol pour obtenir le copolymère poly-(β-méthylaspartate/leucine). On saponifie ensuite les groupes méthylester par la soude N/10 dans le méthanol pour obtenir l'acide poly-(ASP(OH)/leu). L'analyse des acides aminés montre qu'on a une proportion de leucine de 56 % et de 44 % pour l'acide aspartique. Le poids moléculaire $M_n$ est de 35 000 (mesuré par GPC).

On dissout 1 g de poly-(Asp(OH)/leu) dans 20 g de DMF sec. On ajoute 3,25 g de tributylamine (4 équivalents par rapport aux restes Asp(OH)) et 2,64 g de chlorométhylpivalate (4 équivalents). On laisse sous agitation 3 jours à température ambiante. On précipite le polymère dans $H_2O$ distillée, on le redissout dans l'acétone, on précipite dans l'éther de pétrole, on redissout dans l'acétone, on reprécipite avec $H_2O$. On sèche.

L'analyse RMN montre qu'on a bien obtenu du poly-(pivaloyloxyméthylaspartate/leucine) le taux d'estérification étant d'environ 20 % (δ = 7,5, protons benzyliques ; δ = 8 ppm, protons NH ; δ = 5,4 et 4,8, protons alpha-CH ; δ = 3,95, protons $-O-CH_3$ non saponifiés ; δ = 3,3 et 2, protons $-CH_2-$, δ = 1,35 ppm, protons t-butyle). Malgré le taux d'estérification relativement faible, le copolymère est soluble dans l'acétone.

Exemple 15

A titre comparatif, on a étudié les vitesses d'hydrolyse en milieu alcalin d'un copolymère suivant l'invention marqué comme décrit à l'Exemple 7, (copolymère glutamate de pivaloyloxyméthyl-leucine 85-15 suivant l'Exemple 8) et d'un copolymère de polyglutamate de méthyle-leucine 85-15, de l'art antérieur préparé par les techniques connues habituelles au moyen de méthanol marqué au $^{14}C$. Le polymère suivant l'invention (A) a été converti en un film d'environ 0,25 mm par dépôt sur une plaque de verre d'une couche de solution acétonique qu'on a ensuite soumise à évaporation à l'air. Pour le film de contrôle (B)

on a procédé de même à partir d'une solution dans l'acide TFA. On a soigneusement lavé ce dernier film à l'eau afin d'éliminer toute trace de TFA.

On a plongé les films (A) et (B) dans des solutions aqueuses à pH 9,5 (tampon phosphate) en lente agitation et, à intervalles, on a mesuré la radioactivité des produits dissous dans ces solutions d'où on a calculé les taux d'hydrolyse des polymères soumis au test.

On a trouvé les résultats suivants exprimés en % en poids de matière hydrolysée après un temps donné (heures).

| Echantillon (A) | | Echantillon (B) | |
|---|---|---|---|
| temps (h) | % en poids | temps (h) | % en poids |
| 1,5 | 2,8 | 18 | 1,8 |
| 2,5 | 5,0 | 42 | 4,2 |
| 4,5 | 13,2 | 64 | 5 |
| 8 | 37,25 | 88 | 5,9 |
| 8 | dissolution | 112 | 6,8 |

On voit, d'après les résultats ci-dessus que le copolymère (A) se dégrade à tel point en 8-10 h que le médicament qu'il aurait pu contenir se serait dissous entièrement dans de telles conditions, alors que le copolymère de l'art antérieur n'est que très peu altéré après 112 h dans les mêmes conditions.

En faisant varier le taux de leucine du copolymère (A), on peut faire varier le temps d'hydrolyse, dans le sens d'une augmentation en augmentant ledit taux et d'une diminution en réduisant ledit taux.

**Revendications** (pour l'Etat contractant IT)

1. Polypeptide constitué de polyaspartate ou polyglutamate d'acyloxyméthyle ou d'aroyloxyméthyle biodégradable de formule

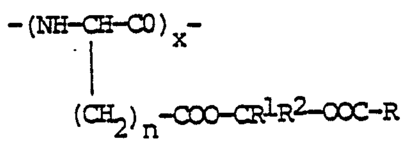

(I)

dans laquelle $R^1$ et $R^2$ sont des groupes alcoyles ou un atome d'hydrogène, R étant un reste aliphatique ou aromatique substitué ou non ; ou bien $R^2$ est un groupe alcoyle où un atome d'hydrogène et R et $R^1$ sont liés l'un à l'autre sous forme d'un pont éthylène ou vinylène substitué ou non ; et ses copolymères avec d'autres acides aminés de formule

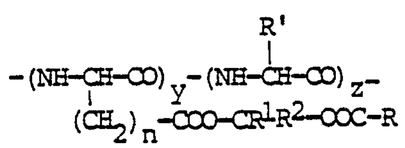

(II)

où les groupes R, $R^1$ et $R^2$ ont le sens donné ci-dessus et où R' est un reste d'amino-acide libre ou partiellement ou totalement estérifié ; n vaut 1 ou 2 et x qui est égal à y + z est choisi pour que la masse moléculaire du polypeptide ne soit pas inférieure à 5 000 D.

2. Polypeptide suivant la revendication 1, caractérisé par le fait que R est choisi parmi les restes méthyle, éthyle, isopropyle, isobutyle, tert-butyle, phényle et benzyle.

3. Polypeptide suivant la revendication 1, caractérisé par le fait que, R et $R^1$ étant liés l'un à l'autre, le maillon formé par ceux-ci est choisi parmi les formules suivantes : $-CH_2-CH_2-$, $-CH=CH-$, $-CH(CH_3)-CH(CH_3)$, $-C(CH_3)=C(CH_3)-$, 1,2-phénylène, cyclohexénylène, cyclopenténylène et cyclopentadiénylène.

4. Polypeptide suivant la revendication 1, caractérisé par le fait que le composé I est choisi parmi les glutamate et aspartate d'acyl- et aroyl-oxyméthyle et que le composé II est choisi parmi les copolymères de glutamate ou aspartate d'acyl- et d'aroyl-oxyméthyle avec un ou plusieurs autres acides aminés choisis parmi l'alanine, la leucine, la valine et la phénylalanine.

5. Polypeptide suivant la revendication 1, caractérisé par le fait que le copolymère II est choisi parmi les copolymères des polyglutamate ou aspartate I avec, respectivement, l'acide glutamique et/ou les glutamates d'alcoyles inférieurs et l'acide aspartique et/ou les aspartates d'alcoyles inférieurs.

6. Polypeptide suivant la revendication 4, caractérisé par le fait que les groupes acyles sont choisis parmi les groupes acétyle, propionyle, butyryle, isobutyryle, pivaloyle, benzoyle et phénylacétyle.

7. Utilisation du polypeptide I et ses copolymères II suivant la revendication 1 comme réservoir de médicament à effet retard dans l'organisme, ce dernier étant progressivement libéré à son lieu de destination consécutivement à la biodégradation du polymère porteur.

8. Utilisation suivant la revendication 7, caractérisée par le fait qu'on mélange ledit médicament et ledit polymère de façon homogène et qu'on façonne ce mélange sous une forme pharmaceutiquement acceptable.

9. Utilisation suivant la revendication 8, caractérisée par le fait qu'on mélange au polymère un polyalcoylène glycol de façon à le plastifier, qu'on y ajoute un médicament, puis qu'on moule par la chaleur le produit thermoplastique ainsi obtenu sous forme de granulés, bâtonnets, capsules ou autres particules aptes à être administrées par toutes voies habituelles.

10. Utilisation des polymères I et copolymères II, suivant la revendication 1, pour fabriquer des implants et prothèses biodégradables utilisables en chirurgie.

11. Procédé de préparation du polypeptide formule I, caractérisé par le fait qu'on fait réagir le polyaminoacide correspondant sous forme de son sel avec une amine tertiaire avec l'ester d'halogénométhyle

$$X—CR^1R^2—OCO—R \qquad (III)$$

où X = Cl, Br ou I et R, $R^1$ et $R^2$ répondent aux définitions précitées, le composé I se formant avec élimination simultanée du sel d'amine de l'acide hydrohalogéné correspondant.

12. Procédé de préparation du copolymère de formule II, caractérisé par le fait qu'on opère comme indiqué à la revendication 11 à partir du copolyaminoacide correspondant.

**Revendications** (pour l'Etat contractant AT)

1. Procédé pour la préparation d'un polypeptide (I) constitué de polyaspartate ou polyglutamate d'acyloxyméthyle ou d'aroyloxyméthyle biodégradable de formule

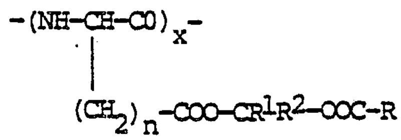

(I)

dans laquelle $R^1$ et $R^2$ sont des groupes alcoyles ou un atome d'hydrogène, R étant un reste aliphatique ou aromatique substitué ou non ; ou bien $R^2$ est un groupe alcoyle où un atome d'hydrogène et R et $R^1$ sont liés l'un à l'autre sous forme d'un pont éthylène ou vinylène substitué ou non ; et ses copolymères avec d'autres acides aminés de formule (II)

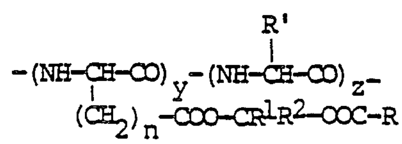

(II)

où les groupes R, $R^1$ et $R^2$ ont le sens donné ci-dessus et où R' est un reste d'amino-acide libre ou partiellement ou totalement estérifié ; n vaut 1 ou 2 et x qui est égal à y + z est choisi pour que la masse moléculaire du polypeptide ne soit pas inférieure à 5 000 D, caractérisé par le fait qu'on fait réagir le polyaminoacide correspondant sous forme de son sel avec une amine tertiaire avec l'ester d'halogénométhyle

$$X—CR^1R^2—OCO—R \qquad (III)$$

où X = Cl, Br ou I et R, $R^1$ et $R^2$ répondent aux définitions précitées, le composé I se formant avec élimination simultanée du sel d'amine de l'acide hydrohalogène correspondant.

2. Procédé suivant la revendication 1, caractérisé par le fait que R est choisi parmi les restes méthyle, éthyle, isopropyle, isobutyle, tert-butyle, phényle et benzyle.

3. Procédé suivant la revendication 1, caractérisé par le fait que, R et $R^1$ étant liés l'un à l'autre, le maillon formé par ceux-ci est choisi parmi les formules suivantes : $—CH_2—CH_2—$, $—CH=CH—$,

$-CH(CH_3)-CH(CH_3)$, $-C(CH_3)=C(CH_3)-$, 1,2-phénylène, cyclohexénylène, cyclopenténylène et cyclopentadiénylène.

4. Procédé suivant la revendication 1, caractérisé par le fait que le composé I est choisi parmi les glutamate et aspartate d'acyl- et aroyl-oxyméthyle et que le composé II est choisi parmi les copolymères de glutamate ou aspartate d'acyl- et d'aroyl-oxyméthyle avec un ou plusieurs autres acides aminés choisis parmi l'alanine, la leucine, la valine et la phénylalaline.

5. Procédé suivant la revendication 1, caractérisé par le fait que le copolymère II est choisi parmi les copolymères des polyglutamate ou aspartate I avec, respectivement, l'acide glutamique et/ou les glutamates d'alcoyles inférieurs et l'acide aspartique et/ou les aspartates d'alcoyles inférieurs.

6. Procédé suivant la revendication 4, caractérisé par le fait que les groupes acyles sont choisis parmi les groupes acétyle, propionyle, butyryle, isobutyryle, pivaloyle, benzoyle et phénylacétyle.

7. Utilisation du polypeptide I et ses copolymères II obtenus suivant la revendication 1 comme réservoir de médicament à effet retard dans l'organisme, ce dernier étant progressivement libéré à son lieu de destination consécutivement à la biodégradation du polymère porteur.

8. Utilisation suivant la revendication 7, caractérisée par le fait qu'on mélange ledit médicament et ledit polymère de façon homogène et qu'on façonne ce mélange sous une forme pharmaceutiquement acceptable.

9. Utilisation suivant la revendication 8, caractérisée par le fait qu'on mélange au polymère un polyalcoylène glycol de façon à le plastifier, qu'on y ajoute un médicament, puis qu'on moule par la chaleur le produit thermoplastique ainsi obtenu sous forme de granulés, bâtonnets, capsules ou autres particules aptes à être administrés par toutes voies habituelles.

10. Utilisation des polymères I et copolymères II, obtenue suivant la revendication 1, pour fabriquer des implants et prothèses biodégradables utilisables en chirurgie.

**Claims** (for the Contracting State IT)

1. Polypeptide comprising biodegradable acyloxymethyl or aroyloxymethyl polyaspartate or polyglutamate with the formula

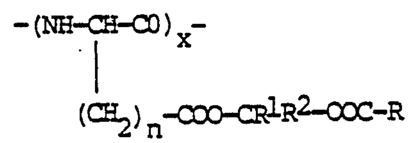

(I)

in wich $R^1$ and $R^2$ are alkyl groups or one atom of hydrogen, R being a substituted or non-substituted aliphatic or aromatic residue ; or $R^2$ is an alkyl group or one atom of hydrogen and R and $R^1$ are interlinked in the form of a substituted or non-substituted ethylene or vinylene bridge ; and its copolymers with other amino-acids with the formula

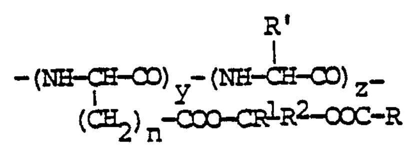

(II)

in which groups R, $R^1$ and $R^2$ have the value given above and where R' is a free or partially or completely esterified amino-acid residue ; the value of n is 1 or 2 and x which is equal to y + z is selected so that the molecular mass of the polypeptide is not lower than 5 000 D.

2. Polypeptide according to claim 1, characterised in that R is selected from methyl, ethyl, isopropyl, isobutyl, tert-butyl, phenyl and benzyl residues.

3. Polypeptide according to claim 1, characterised in that, R and $R^1$ being interconnected, the chain link formed by these is selected from the following formulae : $-CH_2-CH_2-$, $-CH=CH-$, $-CH(CH_3)-CH(CH_3)$, $-C(CH_3)=C(CH_3)-$, 1,2-phenylene, cyclohexenylene, cyclopentenylene, cyclopentadienylene.

4. Polypeptide according to claim 1, characterised in that the compound I is selected from acyl and aryloxymethyl glutamates and aspartates and that compound II is selected from acyl- and aroyloxymethyl glutamate or aspartate copolymers with one or more other amino-acids selected from alanine, leucine, valine and phenylalaline.

5. Polypeptide according to claim 1, characterised in that the copolymer II is selected from the polyglutamate or aspartate copolymers I with, correspondingly, glutamic acid and/or lower alkyl glutamates and aspartic acid and/or lower alkyl aspartates.

6. Polypeptide according to claim 4, characterised in that the acyl groups are selected from acetyl, propionyl, butyryl, isobutyryl, pivaloyl, benzoyl, and phenylacetyl groups.

7. Use of polypeptide I and its copolymers II according to claim 1 as a drug reservoir with a delay effect in the organism, the drug being released progressively to its destination consequent upon the biodegradation of the carrier polymer.

8. Use according to claim 7, characterised in that the said drug and the said polymer are mixed in a homogeneous manner and that the mixture is shaped in a pharmaceutically acceptable form.

9. Use according to claim 8, characterised in that a polyalkylene glycol is mixed into the polymer in order to plasticise it, that a drug is added to it, then the thermoplastic product thus obtained is heat moulded in the form of granules, rods, capsules or other particles suitable for being administered in the usual manner.

10. Use of polymers I and copolymers II, according to claim 1, for manufacturing biodegradable implants and prosthetics which can be used in surgery.

11. Preparation method for formula I polypeptide, characterised in that the corresponding polyaminoacid is made to react in the form of its salt with a tertiary amine with the halogenomethyl ester

$$X—CR^1R^2—OCO—R \qquad (III)$$

where X = Cl, Br or I and R, $R^1$ and $R^2$ correspond to the above-mentioned definitions, the compound I forming with simultaneous elimination of the corresponding amine salt of the hydrohylic acid.

12. Preparation method for formula II copolymer, characterised in that the procedure as indicated in claim 11 starts with the corresponding copolyaminoacid.

**Claims** (for the Contracting State AT)

1. Preparation process of a polypeptide (I) comprising biodegradable acyloxymethyl or aroyloxymethyl polyaspartate or polyglutamate with the formula

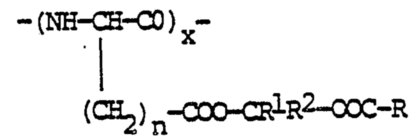

(I)

in which $R^1$ and $R^2$ are alkyl groups or one atom of hydrogen, R being a substituted or non-substituted aliphatic or aromatic residue ; or $R^2$ is an alkyl group or one atom of hydrogen and R and $R^1$ are interlinked in the form of a substituted or non-substituted ethylene or vinylene bridge ; and its copolymers with other amino-acids with the formula (II)

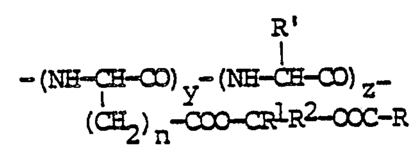

(II)

in which group R, $R^1$ and $R^2$ have the value given above and where R' is a free or partially or completely esterified amino-acid residue ; the value of n is 1 or 2 and x which is equal to y + z is selected so that the molecular mass of the polypeptide is not lower than 5 000 D.

2. Preparation process according to claim 1, characterised in that R is selected from methyl, ethyl, isopropyl, isobutyl, tert-butyl, phenyl and benzyl residues.

3. Preparation process according to claim 1, characterised in that, R and $R^1$ being interconnected, the chain link formed by these is selected from the following formulae : $—CH_2—CH_2—$, —CH=CH—, $—CH(CH_3)—CH(CH_3)$, $—C(CH_3)=C(CH_3)—$, 1.2-phenylene, cyclohexenylene, cyclopentenylene, cyclopentadienylene.

4. Preparation process according to claim 1, characterised in that the compound I is selected from acyl and aryloxymethyl glutamates and aspartates and that compound II is selected from acyl- and aroyloxymethyl glutamate or aspartate copolymers with one or more other amino-acids selected from alanine, leucine, valine and phenylalaline.

5. Preparation process according to claim 1, characterised in that the copolymer II is selected from the polyglutamate or aspartate copolymers I with, correspondingly, glutamic acid and/or lower alkyl glutamates and aspartic acid and/or lower alkyl aspartates.

6. Preparation process according to claim 4, characterised in that the acyl groups are selected from acetyl, propionyl, butyryl, isobutyryl, pivaloyl, benzoyl, and phenylacetyl groups.

7. Use of polypeptide I and its copolymers II according to claim 1 as a drug reservoir with a delay effect in the organism, the drug being released progressively to its destination consequent upon the biodegradation of the carrier polymer.

8. Use according to claim 7, characterised in that the said drug and the said polymer are mixed in a homogeneous manner and that the mixture is shaped in a pharmaceutically acceptable form.

9. Use according to claim 8, characterised in that a polyalkylene glycol is mixed into the polymer in order to plasticise it, that a drug is added to it, then the thermoplastic product thus obtained is heat moulded in the form of granules, rods, capsules or other particles suitable for being administered in the usual manner.

10. Use of polymers I and copolymers II, according to claim 1, for manufacturing biodegradable implants and prosthetics which can be used in surgery.

**Patentansprüche** (für den Vertragsstaat AT)

1. Verfahren zur Herstellung eines Polypeptides (I) aus biologisch abbaubarem Polyaspartat oder Polyglutamat vom Acyloximethyl oder Aroyloximethyl der Formel

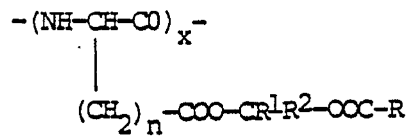

(I)

in der $R^1$ und $R^2$ Alkylgruppen oder ein Wasserstoffatom sind, R ein aliphatischer oder aromatischer, gegebenenfalls substituierter Rest oder $R^2$ eine Alkylgruppe oder ein Wasserstoffatom ist, R und $R^1$ miteinander in Form einer gegebenenfalls substituierten Äthylen- oder Vinylenbrücke verbunden sind ; und seine Copolymere mit anderen Aminsäuren der Formel

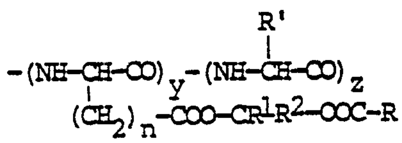

(II)

wo die Gruppen R, $R^1$ und $R^2$ die oben angegebene Bedeutung haben und wo R′ ein freier, partiell oder vollständig veresteter Aminosäurerest ist, n den Wert 1 oder 2 hat und x, das gleich y + z ist, so gewählt ist, daß die Molekularmasse des Peptides nicht unterhalb 5 000 D ist, dadurch gekennzeichnet, daß man die entsprechende Polyaminosäure in Form ihres Salzes mit einem tertiären Amin mit dem Ester von Halogenmethyl

$$X—CR^1R^2—OCO—R \qquad (III)$$

reagieren läßt, wo X = Cl, Br oder J und R, $R^1$ und $R^2$ den obenerwähnten Definitionen entsprechen, wobei die Zusammensetzung I sich mit gleichzeitiger Beseitigung des Aminsalzes der entsprechenden Hydrohalogensäure bildet.

2. Verfahren nach Anspruch 1, dadurch gekennzeichnet, daß R aus den Methyl-, Äthyl-, Isopropyl-, Isobutyl-, Tert-butyl-, Phenyl- und Benzylresten ausgewählt wird.

3. Verfahren nach Anspruch 1, dadurch gekennzeichnet, daß R und $R^1$ miteinander verbunden sind, wobei das durch diese gebildete Glied aus folgenden Formeln gewählt ist : $—CH_2—CH_2—$, $—CH=CH—$, $—CH(CH_3)—CH(CH_3)$, $—C(CH_3)=C(CH_3)—$, 1,2-Phenylen, Cyclohexenylen, Cyclopentenylen und Cyclopentadienylen.

4. Verfahren nach Anspruch 1, dadurch gekennzeichnet, daß die Zusammensetzung I aus den Glutamaten und Aspartaten von Acyl- und Aroyl-oximethyl und die Zusammensetzung II aus den Copolymeren von Glutamat oder Aspartat von Acyl- und Aroyl-oximethyl mit einer oder mehreren anderen Aminosäuren ausgewählt wird, die aus Alanin, Leucin, Valin und Phenylalanin ausgewählt sind.

5. Verfahren nach Anspruch 1, dadurch gekennzeichnet, daß das Copolymer II ausgewählt wird aus den Copolymeren der Polyglutamate bzw. Aspartate I mit Glutaminsäure und/oder den Glutamaten der niederen Alkyle und Asparginsäure und/oder den Aspartaten von niederen Alkylen.

6. Verfahren nach Anspruch 4, dadurch gekennzeichnet, daß die Acylgruppen aus den Acetyl-, Propionyl-, Butyryl-, Isobutyryl-, Pivaloyl-, Benzoyl- und Phenylacetylgruppen ausgewählt sind.

7. Verwendung des Polypeptides I und seiner Copolymere II gemäß Anspruch 1 als Depot eines Arzneimittels mit verzögerter Wirkung im Körper, wobei letzteres schrittweise an seinen Bestimmungsort freigesetzt wird infolge des biologischen Abbaues des Trägerpolymers.

8. Verwendung nach Anspruch 7, dadurch gekennzeichnet, daß man das Arzneimittel und das Polymer homogen vermischt und daß man diese Mischung in eine annehmbare pharmazeutische Form bringt.

9. Verwendung nach Anspruch 8, dadurch gekennzeichnet, daß man dem Polymer ein Polyalkylenglykol derart zumischt, daß es weich wird, daß man diesem ein Arzneimittel hinzufügt, daß man dann in Wärme das so erhaltene, thermoplastische Produkt in Form von Körnchen, Stäbchen, Kapseln oder anderen geeigneten, zu handhabenden Teilchen auf irgendeine übliche Weise mahlt.

10. Verwendung des Polymers I und der Copolymere II nach Anspruch 1 zur Herstellung von in der Chirurgie verwendbaren biologisch abbaubaren Implantaten und Prothesen.

**Patentansprüche** (für den Vertragsstaat IT)

1. Polypeptid aus biologisch abbaubarem Polyaspartat oder Polyglutamat vom Acyloximethyl oder Aroyloximethyl der Formel

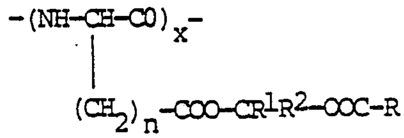

(I)

in der $R^1$ und $R^2$ Alkylgruppen oder ein Wasserstoffatom sind, R ein aliphatischer oder aromatischer, gegebenenfalls substituierter Rest oder $R^2$ eine Alkylgruppe oder ein Wasserstoffatom ist, R und $R^1$ miteinander in Form einer gegebenenfalls substituierten Äthylen- oder Vinylenbrücke verbunden sind ; und seine Copolymere mit anderen Aminsäuren der Formel

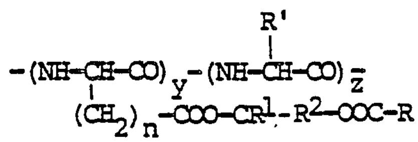

(II)

wo die Gruppen R, $R^1$ und $R^2$ die oben angegebene Bedeutung haben und wo R' ein freier, partiell oder vollständig veresteter Aminosäurerest ist, n den Wert 1 oder 2 hat und x, das gleich y + z ist, so gewählt ist, daß die Molekularmasse des Peptides nicht unterhalb von 5 000 D ist.

2. Polypeptid nach Anspruch 1, dadurch gekennzeichnet, daß R aus den Methyl-, Äthyl-, Isopropyl-, Isobutyl-, Tert-butyl-, Phenyl- und Benzylresten ausgewählt ist.

3. Polypeptid nach Anspruch 1, dadurch gekennzeichnet, daß R und $R^1$ miteinander verbunden sind, wobei das durch diese gebildete Glied aus folgenden Formeln gewählt ist : $—CH_2—CH_2—$, $—CH{=}CH—$, $—CH(CH_3)—CH(CH_3)$, $—C(CH_3){=}C(CH_3)—$, 1,2-Phenylen, Cyclohexenylen, Cyclopentenylen und Cyclopentadien.

4. Polypeptid nach Anspruch 1, dadurch gekennzeichnet, daß die Zusammensetzung I aus den Glutamaten und Aspartaten von Acyl- und Aroyl-oximethyl und die Zusammensetzung II aus den Copolymeren von Glutamat oder Aspartat von Acyl- und Aroyl-oximethyl mit einer oder mehreren anderen Aminosäuren ausgewählt wird, die aus Alanin, Leucin, Valin und Phenylalanin ausgewählt sind.

5. Polypeptid nach Anspruch 1, dadurch gekennzeichnet, daß das Copolymer II ausgewählt wird aus den Copolymeren der Polyglutamate bzw. Aspartate I mit Glutaminsäure und/oder den Glutamaten der niederen Alkyle und Asparginsäure und/oder den Aspartaten von niederen Alkylen.

6. Polypeptid nach Anspruch 4, dadurch gekennzeichnet, daß die Acylgruppen aus den Acetyl-, Propionyl-, Butyryl-, Isobutyryl-, Pivaloyl-, Benzoyl- und Phenylacetylgruppen ausgewählt sind.

7. Verwendung des Polypeptides I und seiner Copolymere II gemäß Anspruch 1 als Depot eines Arzneimittels mit verzögerter Wirkung im Körper, wobei letzteres schrittweise an seinen Bestimmungsort freigesetzt wird infolge des biologischen Abbaues des Trägerpolymers.

8. Verwendung nach Anspruch 7, dadurch gekennzeichnet, daß man das Arzneimittel und das Polymer homogen vermischt und daß man diese Mischung in eine annehmbare pharmazeutische Form bringt.

9. Verwendung nach Anspruch 8, dadurch gekennzeichnet, daß man dem Polymer ein Polyalkylenglykol derart zumischt, daß es weich wird, daß man diesem ein Arzneimittel hinzufügt, daß man dann in Wärme das so erhaltene, thermoplastische Produkt in Form von Körnchen, Stäbchen, Kapseln oder anderen geeigneten, zu handhabenden Teilchen auf irgendeine übliche Weise mahlt.

10. Verwendung des Polymers I und der Copolymere II nach Anspruch 1 zur Herstellung von in der Chirurgie verwendbaren, biologisch abbaubaren Implantaten und Prothesen.

11. Verfahren zur Herstellung des Polypeptides der Formel I, dadurch gekennzeichnet, daß man die entsprechende Polyaminosäure in Form ihres Salzes mit einem tertiären Amin mit dem Ester von Halogenmethyl

$$X—CR^1R^2—OCO—R \qquad (III)$$

reagieren läßt, wo X = Cl, Br oder J und R, $R^1$ und $R^2$ den obenerwähnten Definitionen entsprechen, wobei die Zusammensetzung I sich mit gleichzeitiger Beseitigung des Aminsalzes der entsprechenden Hydrohalogensäure bildet.

12. Verfahren zur Herstellung des Copolymers der Formel II, dadurch gekennzeichnet, daß man, wie im Anspruch 11 ausgehend, von entsprechender Copolyaminsäure vorgeht.